# EUROPEAN PATENT SPECIFICATION

(11) **EP 1 706 103 B1**
(45) Date of publication and mention of the grant of the patent: **10.07.2013**
(21) Application number: 04803101.7
(22) Date of filing: 02.11.2004
(51) Int. Cl.: A61K 9/50, C12N 5/00

(54) **THREE-DIMENSIONAL MAMMALIAN OVARIAN FOLLICULAR CELL AND OVARIAN FOLLICLE CULTURE SYSTEMS IN A BIOCOMPATIBLE MATRIX**
DREIDIMENSIONALE SÄUGETIER-OVARIAL-FOLLIKULARZELL- UND OVARIAL-FOLLIKULARZELL-KULTURSYSTEME IN EINER BIOKOMPATIBLEN MATRIX
PREPARATION DE SYSTEMES CELLULAIRES FOLLICULAIRES OVARIENS MAMMALIENS TRIDIMENSIONNELS ET DE CULTURE DE FOLLICULES OVARIENS D'UNE MATRICE BIOCOMPATIBLE

(30) Priority: 03.11.2003 IT MI20032115
(43) Date of publication of application: 04.10.2006
(73) Proprietor: Universita' degli Studi di Milano, 20122 Milano (IT); Conte, Ubaldo, 27100 Pavia (IT); Torre, Maria Luisa, 27100 Pavia (IT); Universita' degli Studi di Bologna, 40126 Bologna (IT)
(72) Inventor: VIGO, Daniele Università Degli Studi di Milano, 20122 Milano (IT); RUSSO, Vincenzo Università Degli Studi di Milano, 20122 Milano (IT); FAUSTINI, Massimo Univ. Degli Studi di Milano, 20122 Milano (IT); STACCHEZZINI, Simona Univ. Degli Studi di Milano, 20122 Milano (IT); CONTE, Ubaldo Università Degli Studi di Pavia, 27100 Pavia (IT); TORRE, Maria Luisa Univ. Degli Studi di Pavia, 27100 Pavia (IT); ACCORSI, Pier Attilio Univ. Degli Studi di Bologna, 40126 Bologna (IT); GALEATI, Giovanna Univ. Degli Studi di Bologna, 40126 Bologna (IT); SPINACI, Marcella Univ. Degli Studi di Bologna, 40126 Bologna (IT)
(74) Representative: Long, Giorgio
(86) International application number: PCT/EP2004/012384
(87) International publication number: WO 2005/041942

(56) References cited:
- EP-B- 0 922 451
- EP-B1- 0 922 451
- WO-A-00/29206
- WO-A-91/01720
- PANGAS S A ET AL: "Novel approach for the three-dimensional culture of granulosa cell-oocyte complexes" TISSUE ENGINEERING, LARCHMONT, NY, US, vol. 9, no. 5, October 2003 (2003-10), pages 1013-1021, XP002323638 ISSN: 1076-3279
- KREEGER P K ET AL: "A NOVEL THREE-DIMENSIONAL SYSTEM FOR THE IN VITRO CULTURE OF IMMATURE MURINE OVARIAN FOLLICLES" BIOLOGY OF REPRODUCTION, SOCIETY FOR THE STUDY OF REPRODUCTION, CHAMPAIGN, IL, US, vol. 68, no. SUPPL 1, 19 July 2003 (2003-07-19), page 199, XP009046061 ISSN: 0006-3363
- KREEGER PAMELA K ET AL: "Murine granulosa cell morphology and function are regulated by a synthetic Arg-Gly-Asp matrix." MOLECULAR AND CELLULAR ENDOCRINOLOGY, vol. 205, no. 1-2, 31 July 2003 (2003-07-31), pages 1-10, XP002410515 ISSN: 0303-7207
- MARESH G A ET AL: "EFFECTS OF EXTRACELLULAR MATRIX ON THE EXPRESSION OF SPECIFIC OVARIAN PROTEINS" BIOLOGY OF REPRODUCTION, vol. 43, no. 6, 1990, pages 965-976, XP002410516 ISSN: 0006-3363
- HUET C ET AL: "Extracellular matrix regulates ovine granulosa cell survival, proliferation and steroidogenesis: Relationships between cell shape and function" JOURNAL OF ENDOCRINOLOGY, vol. 169, no. 2, May 2001 (2001-05), pages 347-360, XP002410517 ISSN: 0022-0795
- MA HSIAO-LI ET AL: "Chondrogenesis of human mesenchymal stem cells encapsulated in alginate beads." JOURNAL OF BIOMEDICAL MATERIALS RESEARCH, vol. 64A, no. 2, 1 February 2003 (2003-02-01), pages 273-281, XP002410518 ISSN: 0021-9304
- MAGYAR J P ET AL: "MASS PRODUCTION OF EMBRYOID BODIES IN MICROBEADS" ANNALS OF THE NEW YORK ACADEMY OF SCIENCES, NEW YORK ACADEMY OF SCIENCES, NEW YORK, NY, US, vol. 944, 7 October 2001 (2001-10-07), pages 135-143, XP001118571 ISSN: 0077-8923
- WEBER M ET AL: "Formation of cartilage matrix proteins by BMP-transfected murine mesenchymal stem cells encapsulated in a novel class of alginates" BIOMATERIALS, ELSEVIER SCIENCE PUBLISHERS BV., BARKING, GB, vol. 23, no. 9, May 2002 (2002-05), pages 2003-2013, XP004345246 ISSN: 0142-9612
- COULOMBEL L ET AL: "LINEAGE-SPECIFIC AND STAGE-SPECIFIC ADHESION OF HUMAN HEMATOPOIETIC PROGENITOR CELLS TO EXTRACELLULAR MATRICES FROM MARROW FIBROBLASTS" BLOOD, vol. 71, no. 2, 1988, pages 329-334, XP002410519 ISSN: 0006-4971
- LUNDELL B I ET AL: "Activation of beta-1 integrins on CML progenitors reveals cooperation between beta-1 integrins and CD44 in the regulation of adhesion and proliferation" LEUKEMIA (BASINGSTOKE), vol. 11, no. 6, 1997, pages 822-829, XP002410520 ISSN: 0887-6924
- YANAI TAKANORI ET AL: "Separate control of the survival, the self-renewal and the differentiation of hemopoietic stem cells" CELL STRUCTURE AND FUNCTION, vol. 20, no. 2, 1995, pages 117-124, XP002410521 ISSN: 0386-7196
- NEBEL R L ET AL: "MICROENCAPSULATION OF BOVINE SPERMATOZOA FOR USE IN ARTIFICIAL INSEMINATION: A REVIEW" REPRODUCTION, FERTILITY AND DEVELOPMENT, CSIRO, EAST MELBOURNE, AU, vol. 5, no. 6, 1993, pages 701-712, XP000891485 ISSN: 1031-3613
- TORRE M L ET AL: "CALCIUM ALGINATE CAPSULES CONTAINING A HYDROPHILIC POLYMER FOR THE ENCAPSULATION OF SWINE SPERMATOZOA" SCIENCES TECHNIQUES ET PRATIQUES STP PHARMA SCIENCES, PARIS, FR, vol. 8, no. 4, 1998, pages 233-236, XP009073273 ISSN: 1157-1489
- AUTHOR: PECILE A ET AL: "In vitro fertilization of swine oocytes with boar semen encapsulated in barium alginate beads: Preliminary results" THERIOGENOLOGY, LOS ALTOS, CA, US, vol. 53, no. 1, 9 January 2000 (2000-01-09), page 429, XP009073275 ISSN: 0093-691X
- TORRE M L ET AL: "Boar semen controlled delivery system: storage and in vitro spermatozoa release" JOURNAL OF CONTROLLED RELEASE, ELSEVIER, AMSTERDAM, NL, vol. 85, no. 1-3, 13 December 2002 (2002-12-13), pages 83-89, XP004397768 ISSN: 0168-3659
- VIGO DANIELE ET AL: "BOAR SEMEN CONTROLLED-DELIVERY SYSTEM: MORPHOLOGICAL INVESTIGATION AND IN VITRO FERTILIZATION TEST" REPRODUCTION, FERTILITY AND DEVELOPMENT, CSIRO, EAST MELBOURNE, AU, vol. 15, no. 5-6, 2002, pages 307-314, XP009073281 ISSN: 1031-3613
- TORRE M L ET AL: "Boar semen controlled delivery system: analysis of batch seasonal variability" INTERNATIONAL JOURNAL OF PHARMACEUTICS, AMSTERDAM, NL, vol. 242, no. 1-2, 21 August 2002 (2002-08-21), pages 385-387, XP002402011 ISSN: 0378-5173
- TORRE M L ET AL: "Controlled release of swine semen encapsulated in calcium alginate beads" BIOMATERIALS, ELSEVIER SCIENCE PUBLISHERS BV., BARKING, GB, vol. 21, no. 14, July 2000 (2000-07), pages 1493-1498, XP004199070 ISSN: 0142-9612
- COSBY N C ET AL: "MICROENCAPSULATION OF SINGLE, MULTIPLE, AND ZONA PELLUCIDA-FREE MOUSE PREIMPLANTATION EMBRYOS IN SODIUM ALGINATE AND THEIR DEVELOPMENT IN VITRO" JOURNAL OF REPRODUCTION AND FERTILITY, JOURNALS OF REPRODUCTION AND FERTILITY LTD, GB, vol. 90, no. 1, September 1990 (1990-09), pages 19-24, XP001118759 ISSN: 0022-4251
- DE VOS P ET AL: "Effect of the alginate composition on the biocompatibility of alginate-polylysine microcapsules" BIOMATERIALS, ELSEVIER SCIENCE PUBLISHERS BV., BARKING, GB, vol. 18, no. 3, February 1997 (1997-02), pages 273-278, XP004015298 ISSN: 0142-9612
- WANDREY C ET AL: "INFLUENCE OF ALGINATE CHARACTERISTICS ON THE PROPERTIES OF MULTI-COMPONENT MICROCAPSULES" JOURNAL OF MICROENCAPSULATION, TAYLOR AND FRANCIS, BASINGSTOKE, GB, vol. 20, no. 5, September 2003 (2003-09), pages 597-611, XP001170108 ISSN: 0265-2048
- PANGAS STEPHANIE A ET AL: "Novel approach for the three-dimensional culture of granulosa cell-oocyte complexes." TISSUE ENGINEERING, vol. 9, no. 5, October 2003 (2003-10), pages 1013-1021, XP002323638 ISSN: 1076-3279

## Description

### Field of the invention

The present invention relates to semi-permeable membrane capsules containing cells or follicles of various types for the preparation of organs, tissues or biological substances both *in vitro* and *in vivo.*

In recent years there has been great interest in the study of novel technologies suitable for the encapsulation within semi-permeable and biocompatible living cell membranes, with the aim of transplanting cells, tissues or tissue parts into living organisms without resorting to the use of immunosuppressant drugs (Uludag et al., 2000). Currently, the culture of isolated living cells is performed predominantly in liquid media or on mono-layers on suitable culture dishes whilst maintaining appropriate conditions of temperature and humidity. Both the above methods may only simulate, in a very limited manner, the complexity of an entire organism since the cells are deprived of their tissue specific extracellular matrix. During culture, in the absence of extracellular matrix, the cells frequently undergo alterations to their morphology and their biochemical and functional properties, above all due to the adhesion of the cells to an unsuitable substrate, an inadequate supply of nutrients and two dimensional growth conditions. (Sittinger et al., 1996). In their natural environment, cells are found in a complex three-dimensional system constituted by an intricate network of proteins and polysaccharides which plays a dynamic role in the regulation of cellular functionality (Li, 1998). Hence, in order to achieve the development of cells or tissues *in vitro,* the formation of an extracellular matrix, as close to that found physiologically, allowing the three-dimensional organisation of the cells, is indispensable. Such an arrangement, potentially similar to that found in living tissues is able to obviate the aggregation of the cells into dense clumps with the consequent loss of efficiency and functionality.

Many authors have used different types of polymer-based matrices (scaffolds), in order to allow the development of isolated cells *in vitro.* Such matrices have high porosity and are able to provide attachment sites suitable for the orientation and growth of a sufficient number of cells, so as to guarantee survival and functionality, similar to that found *in vivo* (Shapiro and Cohen, 1997). In order to achieve adequate growth of the cells, the structural uniformity of the polymeric scaffold, which must be constituted by biocompatible materials with appropriate mechanical characteristics (Kuo and Ma, 2001) is necessary.

A different approach for the attainment of three dimensional culture systems is the encapsulation of cells, by entrapping a population of living cells inside an artificial extracellular matrix bounded by semi-permeable membranes, thus physically isolating them from the external environment. The extracellular matrix within the capsule is essential so that the cells auto-organise themselves into structures functionally similar to tissues *in vivo.*

It was Chang who succeeded in obtaining "artificial cells": systems constituted by polymeric materials, suitable for encapsulating proteins, enzymes or cells (Chang, 1964). One of the first applications has been the vehicularisation of pancreatic cells in alginate capsules for the treatment of diabetes (Lim and Sun, 1980). Cells or tissues were suspended in sodium alginate, and such suspension was extruded into a solution containing bivalent cations, such as calcium ions: the ions bring about the polymerisation of the polymer and the transformation of the suspension into a rigid matrix (bead). Through subsequent treatment with a solution of poly-L-lysine, a permanent semi-permeable membrane forms on the surface of the capsules, the porosity of which could be adjusted depending on the molecular weight and concentration of the poly-L-lysine, and depending on the concentration and type of alginate used (De Vos et al., 1993).

Recently, Mauchamp *et al*. (1998) have found that isolated porcine thyroid follicular cells organise themselves into pseudofollicles if they are allowed to adhere onto a type-I collagen matrix. Such structures are not obtained with cell cultures in monolayers.

Adequate permeability of the polymeric membrane (cut off) is indispensable for the survival and auto-organisation of encapsulated living cells. The ideal membrane should allow the entry of molecules essential for the survival of the cells and the elimination of secreted substances and the waste substances from cellular metabolism (Colton, 1996); further, it should result in a state of immuno-isolation, inhibiting the entry of effectors of the organism's immune response into the cellular environment.

Semi-permeable membranes, with precise molecular cut-offs, allow the diffusion of cellular secretions, catabolites and metabolites. The permeability and selectivity of the membranes thus represent a first critical aspect in the development of such types of systems. Adequate mechanical properties for the capsules, both in terms of resistance to breakage, and in terms of elasticity, size distribution and surface properties are indispensable.

Primordial ovarian follicles are structures characterised by a single layer of flat cells, similar to epithelial cells: such cells, during the maturation of the follicles, become cuboidal in shape and begin to divide, differentiating into outer theca cells, inner theca and the granulosa. During the entire *in vivo* maturation period giving rise to the Graaf follicle, the granulosa cells are able to produce predominantly oestrogens through aromatase enzyme system, which uses androgens and progesterone as substrates. Following the ovulation process, the granulosa cells differentiate morphologically and functionally moving towards progesterone biosynthesis.

Recently, a novel living cell encapsulation technology, particularly for porcine spermatozoa (EP0922451), has been developed. Divalent ions are added to the seminal material and such suspension is extruded into an aqueous solution of sodium alginate. Upon contact with the alginate solution the divalent ions diffuse towards the outer surface thus inducing the gelification of the alginate around the cellular suspension. Such capsules may have their outer surfaces cross-linked using polyamines, such as for example protamine, thus altering the mechanical properties and the permeability of the membrane.

The advantage of this technology with respect to other encapsulation and micro-encapsulation technologies is that the process steps are reduced and the cells thus contained do not undergo any chemical or physical stresses which would compromise their functionality and structure.

### Detailed description of the invention

To date, no attempts have been reported in the literature of the encapsulation of mammalian ovarian follicular cells or ovarian follicles. The use and culture of ovarian follicles and granulosa cells of bovines, equines, caprines, porcines, canines, felines, lagomorphs, mouse and rat and laboratory species in general, as well as humans, but preferably porcines and bovines, is particularly interesting in that such cells, when suitably cultivated, produce hormones or proteins and/or biologically active substances analogously to those which said cells are able to produce *in vivo*. These physiologically produced substances contribute towards the maturation of the oocyte.

The present disclosure relates to an encapsulation technology for ovarian follicular cells, mature and immature gametes, embryos and ovarian follicles at various stages of mammalian development in a biocompatible matrix, enclosed within a membrane of a divalent or trivalent metal salt of alginic acid, optionally cross-linked on the inner and/or outer surface and/or on both surfaces. Besides the aforementioned cellular species, stem cells of various origins may be vehicularised within the capsules; indeed, the latter show morphological and functional characteristics similar to the granulosa cells which constitute the primordial follicles. Further, genetically modified male and female somatic cells may be vehicularised within the capsules, for example pancreatic and thyroidal cells. Cells, tissue or organ parts, tissues or organs, non-human gametes or embryos may be preserved whilst awaiting encapsulation at laboratory temperature, or by refrigeration, freezing, cryopreservation or lyophilisation.

The cells vehicularised within the capsules auto-arrange themselves *in vitro* into three-dimensional follicular, parenchymatose or alveolar structures, which permit the *in vitro* growth of tissues and multicellular structures functionally similar to the organs within the whole organism.

Said cellular structures express biological functions which may not be currently reproduced *in vitro* with other cell culture technologies. The capsule structure allows the attainment of a microenvironment similar to that found physiologically, characterised by the presence of an extracellular matrix and a semi-permeable membrane which acts as a basal membrane.

The cell cultures obtained using this methodology are useful for the production of peptides, proteins, hormones, for the biological assay of drugs, hormones and hormone precursors, for the evaluation of the efficacy of drugs and the toxicity and teratogenicity of chemical and pharmacological substances, for improving the *in vitro* yields of oocell, follicle and embryo cultures and co-cultures in experimental practices and reproductive biotechnology applications. Further, such cell cultures may be implanted into individuals as hormonal-type replacement therapies, indeed the polymeric film (*i.e*. the membrane coating the capsule) which surrounds the artificial tissue, vehicularised within the capsule, constitutes an immuno-protective barrier which allows the obviation of the use of immunosuppressive drugs.

Particularly, the encapsulated mammalian ovarian follicular cells and ovarian follicles are capable of producing progesterone (P4) and 17β-oestradiol (E2) analogously to that which occurs *in vivo.*

The capsules are essentially constituted by:
- a nucleus containing mammalian stem cells, ovarian follicular cells, gametes and embryos or ovarian follicles and/or a biocompatible and/or biodegradable polymer;
- by a semi-permeable membrane constituted by a divalent or trivalent metal salt of a biocompatible and biodegradable polymer such as for example alginic acid, optionally cross-linked on its inner and/or outer surface and/or on both surfaces, optionally vehicularising a second cellular type.

Within said nucleus the cells are suspended in a gelatinous medium.

The organs or tissues are removed from various mammalian species, such as bovines, equines, caprines, lagomorphs, porcines, canines, felines, rodents and possibly humans, but preferably from porcines and bovines. Such removal may be carried out at the time of slaughter, during the removal of biopsy material or whilst performing surgical operations, but for livestock preferably at the time of normal slaughter. The tissues or organs of interest are removed, preferably the female gonads.

In the case where the organs of interest are the ovaries, these are appropriately removed and washed in a physiological solution, as known to experts of the art.

The somatic cells within the follicle and the gametes are isolated from the tissues by aspiration, centrifugation of the follicular liquids, or digestion of the intracellular matrix as known to those skilled in the art. Following centrifugation, the cellular sediment is washed by repeated passages in culture medium and recovered by removal of the supernatant. The cellular concentration of the sediment is determined by direct counting using a Makler chamber, or Bürker chamber, or by cytofluorimetry, or by using semi-automated and automated cell counters.

The isolated cells may be suspended in culture or maintenance media until their encapsulation, preserving them in an environment at a temperature between room temperature and - 200°C and humidity between 40% and 100%, as known to those skilled in the art.

As culture or maintenance media, the followings may be used: physiological solution (isotonic saline), glucosate solution, Basal Medium Eagle (BME) and derivatives thereof, Hanks salts solution and derivatives thereof, tissue culture medium 199 (TCM 199) and derivatives thereof, phosphate buffered saline (PBS) and derivatives thereof, Krebs salts solution and derivatives thereof, Dulbecco modified Eagle's medium (DMEM) and derivatives thereof, tris-buffered medium (TBM) and derivatives thereof, Tyrode's salts solution and derivatives thereof, Modified sperm washing medium, modified human tubal fluid, Modified ham's F-10 medium, Upgraded B2 INRA medium, B2 INRA Menezo Medium, Upgraded B9 medium and various other culture media specifically used by those skilled in the art, but preferably TCM 199 and derivatives thereof as well known to any specialist skilled in the art.

According to the present invention, the cells, suspended in culture medium or follicular liquid, may be optionally diluted into a culture medium containing a hydrophilic polymer which constitutes the artificial extracellular matrix. The cellular sediment dilution-polymeric solution ratio may be between 1:0.05 and 1:200, and preferably between 1:0.1 to 1:100.

The polymeric material constituting the artificial extracellular matrix of the nucleus of the capsules, forming the subject of the present invention is preferably selected from the group constituted by: glucans, scleroglucans, mannans, galactomannans, gellans, carrageenans, pectins, polyanhydrides, polyaminoacids, polyamines, xanthans, celluloses and derivatives thereof, carboxymethylcellulose, ethylcellulose, methylcellulose, hydroxypropylcellulose hydroxypropylmethylcellulose, polyvinylalcohols, carboxyvinylpolymers, starches, collagens, chitins, chitosans, alginic acid, hyaluronic acid. Such polymers, in aqueous solution, at an appropriate pH value, which depends on the nature of the polymer, as known to those skilled in the art, are generally used in concentrations between 0.01% and 90% of total capsule weight, but preferably between 0.5% and 50%. Preferably, xanthan gum at various viscosities, generally between 800 cP and 1200 cP, is used as artificial extracellular matrix.

The capsule membrane, forming the subject of the present invention, is generally constituted by alginates of divalent metals such as calcium, barium, strontium, zinc and trivalent metals such as aluminium, iron and chromium.

In the preparation of the capsules, forming the subject of the present invention, to the cell suspension is added a divalent or trivalent ion, such ion is added, preferably as a chloride or sulphate in solution, so as to obtain cation concentrations of between 1 and 500 mmol/l and preferably between 5 and 200 mmol/l. The extruded cellular suspension and the alginate solution volume ratio may be between 1:1 and 1:250, and preferably between 1:15 and 1:50.

The cellular suspension is subsequently extruded through extruders, orifices, nozzles or needles, having dimensions between 50µm and 5000µm, preferably through needles having internal diameters between 300µm and 2000µm into a solution of sodium alginate in medium, whilst kept stirring, at speeds between 10 and 200 rpm, but preferably between 20 and 100 rpm. The alginates used in the preparation of the capsules forming the subject of the present invention have, in a 2% solution in water, a viscosity between 200 cP and 20000 cP at 25°C. The alginate concentration in the solutions is between 0.01% and 5% w/v, but preferably between 0.1% and 1%.

The presence of divalent and trivalent ions in the extruded cellular suspension induces the gelification of the alginate at the droplet interface and the formation of a gelatinous membrane with the consequent attainment of the capsule.

Such operations are performed at temperatures between 5°C and 40°C, and preferably at 20-30°C; extrusion occurs using automated or semi-automated microencapsulators, peristaltic or piston pumps or alternatives, or using a manually operated syringe, or appropriate system, at such a speed as to produce between 10 to 250 drops/minute, and preferably 60 drops/minute.

Said capsules may be subjected to cross-linking, by interfacial polymerisation of the alginate using polyamine based cross-linking agents, such as for example: protamine sulphate or phosphate, preferably in solution at concentrations between 0.01% and 5% w/v, or poly-L-lysine bromohydrate having a molecular weight between 1000 Da and 800000 Da in solution at a concentration preferably between 0.01% and 5% w/v, or polyvinylamine at a concentration of from 0.01% to 5% w/v, or chitosans of molecular weight between 15000 Da and 1,000,000 Da in concentrations between 0.01% and 5% w/v.

The cross-linking reaction is carried out at a temperature between 5°C and 40°C, and preferably at 25°C for times between 1 minute and 120 minutes, preferably between 3 and 30 minutes. This procedure causes the conversion of the gelatinous membrane into a semi-permeable rigid membrane of cross-linked alginate. Said capsules have a cross-linked membrane and are recovered by filtration, washed and suspended in an appropriate maintenance medium as known to those skilled in the art.

Spheroidal shaped capsules are obtained having dimensions between 0.5 and 30 mm, but preferably between 2 mm and 10 mm, with membrane thicknesses between 300 µm and 5000 µm. The weight of the capsule produced is between 5 mg and 200 mg, bur preferably between 20mg and 100 mg.

Said capsules, suspended in medium, are preserved at temperatures between -200°C and 40°C but preferably between 4°C and 40°C, and still more preferably at 38.5°C, optionally in a controlled atmosphere, as known to those skilled in the art.

Using disposable, preset and pre-packaged instrumentation, for single and/or multiple preparations, capsules may be prepared by starting from previously prepared, pre-measured and pre-packaged raw materials.

Hence, further further disclosed is a kit for the preparation of capsules, according to the invention, comprising previously prepared, pre-measured and pre-packaged raw material, as well as the relevant disposable, sterile, non sterile or sterilisable materials. The preparation of the capsules may be performed by vehicularising into said capsules: cells, tissues, tissue parts, organs, organ parts, cell cores, gametes and embryos, freshly removed and/or appropriately preserved according to the techniques known to those skilled in the art.

Capsules containing cell cores, tissues, organs or parts thereof, gametes or embryos may be co-incubated, in an appropriate culture medium, with other cell cores, tissues, organs or parts thereof, gametes and embryos, thus encouraging the development of cell cores, tissues, organs or parts thereof, gametes and embryos under conditions simulating the physiological environment.

The biosynthesis of specific products and/or specific biologically active substances is favoured under such conditions. The incubation and/or the co-incubation allow the encapsulated biological structures to produce hormones, metabolites, catabolites and other biologically active substances.

The metabolites, catabolites and the biologically active substances produced or secreted and synthesised by the encapsulated structures may be recovered from the culture medium and/or from within the capsules by aspiration, or removed using techniques known to those skilled in the art.

Said metabolic, catabolic and secretory products may be extracted, purified and appropriately characterised as known to those skilled in the art, without irreversibly damaging the three-dimensional capsular culture system.

Said products may be used directly or following purification or concentration, in order to modulate growth, development, maturation and functionality, of other cells, tissues, organs, gametes and embryos, in other *in vitro* culture systems and/or in *ex vivo,* and/or *in vivo* systems.

Analogously, according to the known art, cell cores, autologous or heterologous tissues or organs or parts thereof, as well as gametes and embryos at various stages of development, may be injected, microinjected, inserted within the capsules, without irreversibly damaging the three-dimensional capsular culture system.

Cell cores, tissues or organs or parts thereof, gametes and embryos may be aspirated or removed from said capsules at pre-arranged times using means and techniques known to those skilled in the art, without irreversibly damaging the three-dimensional capsular culture system. The following examples are reported for the purpose of non-limiting illustration of the capsule preparation process forming the subject of the present invention.

### Example 1: encapsulation and three-dimensional culture of bovine granulosa cells

### 1a) Preparation of the cells

The ovaries at various stages of the oestrous cycle are removed from cows, starting from 16-18 months of age, during normal slaughter, washed with physiological solution at 30°C, as known to those skilled in the art. Follicles having a diameter of 2-6 mm are identified in the ovaries, from which the follicular liquids, containing the granulosa cells, are aspirated using syringes. The cellular suspensions thus obtained are centrifuged and washed twice with 10 ml of TCM199 medium + 10% foetal calf serum + 1% penicillin/streptomycin. Following centrifugation, a cellular sediment is obtained, the cellular concentration of which is determined by direct counting using a Makler chamber.

### 1b) Encapsulation

The cellular sediment is diluted in a solution of xanthan gum (Satiaxane^{®}, SKW Biosystems, France) at 0.5% in TCM199 culture medium containing Earle salts, L-glutamine and sodium bicarbonate (Sigma-Aldrich,); the cellular sediment to xanthan gum solution volume ratio is 1:3. A cellular suspension is obtained, to which is then added a saturated barium chloride solution up to a final concentration of 20 mmol/l of barium ions. The resulting suspension is extruded through needles (26GX1/2", 0.45X13mm) into a medium viscosity (3500 cP,) sodium alginate solution at 0.5% w/v in culture medium, kept stirring using a magnetic stirrer (30 rpm). The cellular suspension to sodium alginate solution volume ratio is 1:25. The extrusion takes place dropwise through the syringe, at a temperature of 25°C. The barium ions react with the sodium alginate forming a barium alginate membrane at the interface of the individual drops of extrudate within 30'. Capsules are obtained, which are collected by filtration, washed twice with culture medium and suspended in an aliquot of the same. Said capsules are subsequently cross-linked on their external surfaces using a 1% solution of protamine sulphate (Sigma-Aldrich, Milan, Italy) in TCM199 culture medium containing Earle salts, L-glutamine and sodium bicarbonate (Sigma-Aldrich,) for 30 minutes at a temperature of 25°C.

The population of granulosa cells is found inside the cross-linked capsule, in an artificial extracellular matrix.

Spheroidal shaped capsules are obtained having dimensions between 2 mm and 10 mm and weights between 20 mg and 100 mg. The capsules thus produced may be preserved, under normal laboratory conditions, in specific controlled environment incubators, by lyophilisation, refrigeration, freezing or cryopreservation.

### 1c) Three-dimensional cell culture

A capsule is placed in a sterile cell culture plate well suspended in 600µl of culture medium (TCM199 containing foetal calf serum (10%), penicillin/streptomycin (1%) and 3-17androstenedione (100 ng/µl).

The plates containing the capsules are maintained in an incubator for 6 days at 38.5°C, 5% CO₂ and 90% humidity.

Every 48 hours, from each well, samples of the medium containing the cellular metabolic products are taken; the samples are promptly frozen in Eppendorf tubes, at a temperature of less than -20°C.

In the wells containing the capsules, the culture medium is substituted with an equal volume of fresh medium, with the continuation of the culture on the same sample.

Hence, the steroidogenic activity, in terms of the production of progesterone (P4) and 17β-oestradiol (E2), has been evaluated in each sample of medium removed from the wells by radioimmuno assay (RIA).

The results are expressed as the ratio between P4 and E2, known to those skilled in the art as the luteinisation index.

**Table 1: Luteinisation index (P4/E2), standard deviation and sample number of the bovine granulosa cells cultivated in the capsules.**

| Days | Mean | Std. Dev. | N |
|---|---|---|---|
| 2 | 5,1 | 32,0 | 39 |
| 4 | 567,9 | 2245,3 | 35 |
| 6 | 9452,5 | 18254,4 | 23 |

From the results reported in table 1 it is deduced that cellular vitality is maintained in the encapsulated cells, with the production of both hormones throughout the entire culture period: The encapsulated cells produce low quantities of progesterone as observed *in vivo* in the follicle prior to ovulation.

That indicates reduced luteinisation of the encapsulated cells, which can only occur in follicular structures very similar to those found *in vivo.*

The information derived from analysis of the results underline that the bovine granulosa cells, encapsulated according to the process of the present invention, have steroidal activity analogous to that in *vivo* and obtainable only with a three-dimensional type cell culture process.

### Reference example 1

In parallel, cell culture has been carried out in monolayers, in this case also evaluating the steroidogenic activity in terms of the production of progesterone (P4) and 17β-oestradiol (E2); the concentrations of such hormones in the samples of medium withdrawn from the wells have been evaluated using radioimmuno assay (RIA).

Non-encapsulated cells are seeded and cultivated in monolayers in welled plates, each containing 600 µl of the culture medium also used for the culture of the encapsulated cells.

Analogously to that described for the encapsulated cells, the plates containing the cells in monolayers are maintained in an incubator for 6 days at 38.5°C, 5% CO₂ and 90% humidity.

Every 48 hours, from each well, samples of the medium containing the cellular metabolic products are taken; the samples are frozen in Eppendorf tubes, at a temperature of less than -20°C. From the wells containing the cells cultivated in monolayers, the culture medium is completely removed and substituted with fresh medium, with the continuation of the culture on the same sample. The results obtained are reported in table 2.

**Table 2: Luteinisation index (P4/E2), standard deviation and sample number of the bovine granulosa cells cultivated in monolayers.**

| Days | Mean | Std. Dev. | N |
|---|---|---|---|
| 2 | 7,4 | 38,5 | 27 |
| 4 | 1700,0 | 3870,9 | 23 |
| 6 | 70201,0 | 131436,5 | 11 |

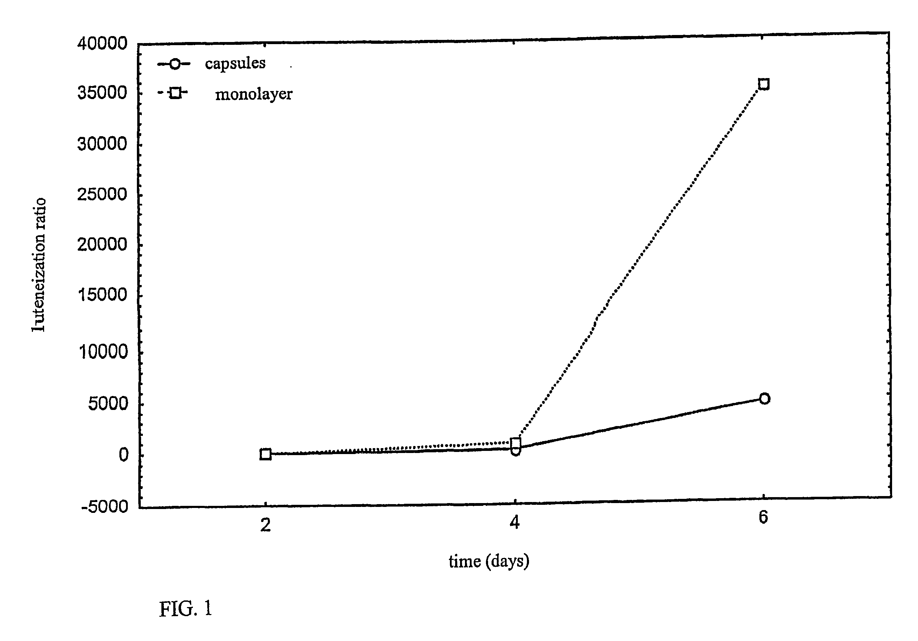

In figure 1 are reported the luteinisation indices of the bovine granulosa cells cultivated in monolayers and in the capsules as a function of culture time.

From the results reported in figure 1 it may be deduced that cellular vitality is maintained with both culture techniques, with the production of both hormones for the entire culture period.

Regarding the progesterone synthesised by the cells cultivated in monolayers, a significant increase in its concentration is observed on the 6^{th} day of culture: this increase is an indication of marked cellular luteinisation.

Such increase is less evident for the encapsulated cells which produce low quantities of progesterone as observed *in vivo* in the follicle prior to ovulation.

That indicates reduced luteinisation of the encapsulated cells and can only occur in follicular-like structures very similar to those found *in vivo*.

### Example 2: encapsulation and three-dimensional culture of porcine granulosa cells

### 2a) Preparation of the cells

The ovaries at various stages of the oestrous cycle are removed from subjects, starting from 6-11 months of age, during normal slaughter, washed with physiological solution at 30°C, as known to those skilled in the art. Follicles having a diameter of 2-6 mm are identified in the ovaries, from which the follicular liquids, containing the granulosa cells, are aspirated using syringes. The cellular suspensions thus obtained are centrifuged and washed twice with 10 ml of TCM199 medium + 10% foetal calf serum + 1% penicillin/streptomycin. Following centrifugation, a cellular sediment is obtained, the cellular concentration of which is determined by direct counting using a Makler chamber.

### 2b) Encapsulation

The cellular sediment is diluted in a solution of xanthan gum (Satiaxane^{®}, SKW Biosystems, France) at 0.5% in TCM199 culture medium containing Earle salts, L-glutamine and sodium bicarbonate (Sigma-Aldrich,); the cellular sediment to xanthan gum solution volume ratio is 1:3. A cellular suspension is obtained, to which is then added a saturated barium chloride solution up to a final concentration of 20 mmol/l of barium ions. The resulting suspension is extruded through needles (26GX1/2", 0.45X13mm) into a medium viscosity (3500 cP,) sodium alginate solution at 0.5% w/v in culture medium, kept stirring using a magnetic stirrer (30 rpm). The cellular suspension to sodium alginate solution volume ratio is 1:25. The extrusion takes place dropwise through the syringe, at a temperature of 25°C. The barium ions react with the sodium alginate forming a barium alginate membrane at the interface of the individual drops of extrudate within 30'. Capsules are obtained, which are collected by filtration, washed twice with culture medium and suspended in an aliquot of the same. Said capsules are subsequently cross-linked on their external surfaces using a 1% solution of protamine sulphate (Sigma-Aldrich, Milan, Italy) in TCM199 culture medium containing Earle salts, L-glutamine and sodium bicarbonate (Sigma-Aldrich,) for 30 minutes at a temperature of 25°C.

The population of granulosa cells is found inside the cross-linked capsule, in an artificial extracellular matrix.

Spheroidal shaped capsules are obtained having dimensions between 2 mm and 10 mm and weights between 20 mg and 100 mg. The capsules thus produced may be preserved, under normal laboratory conditions, in specific controlled environment incubators, by lyophilisation, refrigeration, freezing or cryopreservation.

### 2c) Three-dimensional cell culture

A capsule is placed in a sterile cell culture plate well suspended in 600µl of culture medium (TCM199 containing foetal calf serum (10%), penicillin/streptomycin (1%) and 3-17androstenedione (100 ng/µl)).

The plates containing the capsules are maintained in an incubator for 6 days at 38.5°C, 5% CO₂ and 90% humidity.

Every 48 hours, from each well, samples of the medium containing the cellular metabolic products are taken; the samples are promptly frozen in Eppendorf tubes, at a temperature of less than -20°C.

In the wells containing the capsules, the culture medium is substituted with an equal volume of fresh medium, with the continuation of the culture on the same sample.

Hence, the steroidogenic activity, in terms of the production of progesterone (P4) and 17β-oestradiol (E2), has been evaluated in each sample of medium removed from the wells by radioimmuno assay (RIA).

The results are expressed as the ratio between P4 and E2, known to those skilled in the art as the luteinisation index.

**Table 3: Luteinisation index (P4/E2), standard deviation and sample number of the porcine granulosa cells cultivated in the capsules.**

| Days | Mean | Std. Dev. | N |
|---|---|---|---|
| 2 | 14,8 | 55,3 | 38 |
| 4 | 124,0 | 338,2 | 30 |
| 6 | 43,7 | 83,8 | 20 |

From the results reported in table 3 it may be deduced that cellular vitality is maintained in the encapsulated cells, with the production of both hormones throughout the entire culture period: The encapsulated cells produce low quantities of progesterone as observed *in vivo* in the follicle prior to ovulation.

That indicates reduced luteinisation of the encapsulated cells, which can only occur in follicular structures very similar to those found *in vivo.*

### Reference example 2

In parallel, cell culture has been carried out in monolayers, in this case also evaluating the steroidogenic activity in terms of the production of progesterone (P4) and 17β-oestradiol (E2); the concentrations of such hormones in the samples of medium withdrawn from the wells have been evaluated using radioimmuno assay (RIA).

Non-encapsulated cells are seeded and cultivated in monolayers in welled plates, each containing 600 µl of the culture medium also used for the culture of the encapsulated cells. Analogously to that described for the encapsulated cells, the plates containing the cells in monolayers are maintained in an incubator for 6 days at 38.5°C, 5% CO₂ and 90% humidity.

Every 48 hours, from each well, samples of the medium containing the cellular metabolic products are taken; the samples are frozen in Eppendorf tubes, at a temperature of less than -20°C. From the wells containing the cells cultivated in monolayers, the culture medium is completely removed and substituted with fresh medium, with the continuation of the culture on the same sample. The results obtained are reported in table 4.

**Table 4: Luteinisation index (P4/E2), standard deviation and sample number of the porcine granulosa cells cultivated in monolayers.**

| Days | Mean | Std. Dev. | N |
|---|---|---|---|
| 2 | 2,5 | 2,3 | 36 |
| 4 | 22,1 | 23,4 | 25 |
| 6 | 2160,9 | 4997,9 | 24 |

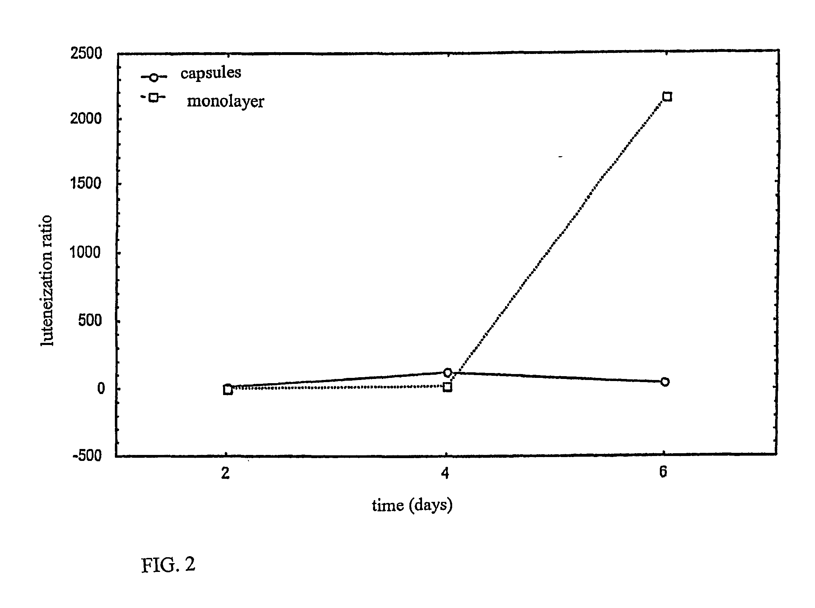

In figure 2 are reported the luteinisation indices of the porcine granulosa cells cultivated in monolayers and in the capsules as a function of culture time.

The information derived from analysis of the results underline that the porcine granulosa cells, encapsulated according to the process of the present invention, have steroidal activity analogous to that *in vivo* and obtainable only with a thee-dimensional type cell culture process.

## Claims

1. Capsules comprising an outer membrane of a divalent or trivalent metal ion salt of alginic acid, and an internal nucleus comprising a suspension of non-human immature female gametes and a biocompatible and/or biodegradable hydrophilic polymer, constituting an artificial extracellular matrix, **characterized in that** the hydrophilic polymer is xanthan gum having viscosity between 800 and 1200 cP and **in that** the alginate membrane contains ovarian follicular cells.

2. The capsules according to claim 1, wherein said cells are suspended in a gelatinous medium.

3. The capsules according to claims 1 or 2 **characterised in that** said non-human immature female gametes and said ovarian follicular cells are auto-organised *in vitro* into three-dimensional follicular type structures, which allow the *in vitro* growth of tissues and multicellular structures functionally similar to organs present in the whole mammalian organism.

4. The capsules according to any of the claims 1 to 3, **characterised in that** said non-human immature female gametes are functionally similar to the organs found in the whole organism and able to synthesise and secrete hormones such as progesterone (P4) and 17β-oestradiol (E2) and other biologically active substances in quantities similar to that which said structures produce *in vivo.*

5. The capsules according to any of the claims 1 to 4 wherein said alginate membrane is gelatinous, bioerodible and is cross-linked on the inner and/or outer surfaces and/or on both surfaces.

6. The capsules according to any of the claims 1 to 5 **characterised in that** the alginate membrane may be cross-linked using cross-linking agents selected from: protamine sulphate or phosphate, poly-L-lysine bromohydrate, polyvinylamine, or chitosans.

7. The capsules according to any of the claims 1 to 6 wherein the outer membrane is constituted by divalent metal alginates selected from: calcium, barium, strontium, zinc or trivalent metals selected from: aluminium, iron, chromium.

8. The capsules according to claim 1, **characterised in that** said xanthan gum, in aqueous solution, is present in concentrations between 0.01% and 90% of the total capsule weight.

9. The capsules according to claim 8, **characterised in that** xanthan gum, in aqueous solution, is present in concentrations between 0.5% and 50% of the total capsule weight.

10. The capsules according to any of the claims 1 to 9 **characterised in that** said capsules have diameters between 0.5 mm and 30 mm, with membrane thicknesses between 300 µm and 5000 µm.

11. The capsules according to claim 10 **characterised in that** said capsules preferably have diameters between 2 mm and 10 mm.

12. The capsules according to any of the claims 1 to 11 **characterised in that** said capsules weigh between 5 mg and 200 mg.

13. The capsules according to claim 12 **characterised in that** said capsules preferably weigh between 20 mg and 100 mg.

14. A process for the preparation of capsules according to any of the claims 1 to 13 which comprises the following steps:
a) suspending non-human immature female gametes in a culture medium or in an appropriate biological liquid containing a biocompatible and/or biodegradable hydrophilic polymer, said polymer being xanthan gum;
b) adding to the suspension thus obtained a divalent or trivalent ion salt until the attainment of ion concentrations between 1 and 500 mmol/l;
c) extruding the cellular suspension through extruders, orifices, nozzles or needles having dimensions between 50 µm and 5000 µm into an alkaline metal alginate solution in culture medium, having a concentration between 0.01% and 5% w/v, kept stirring at a speed between 10 and 200 rpm;
d) vehicularising within the alginate membrane obtained in step c) a second cellular species consisting in ovarian follicular cells either freshly removed or appropriately preserved;
e) optionally, cross-linking the capsules thus formed, through interfacial polymerisation of the alginate using the cross-linking agents according to claim 5, at a temperature between 5° C and 40° C for a time between 1 minute and 120 minutes.

15. The process according to claim 14 further comprising the recovery stage of the capsules by filtration, the washing of the same and their suspension in culture medium.

16. The process according to claim 14 further comprising the preservation stage of the capsules under laboratory culture conditions, or by lyophilisation, refrigeration, freezing or cryopreservation.

17. The process according to claim 14 wherein said vehicularisation stage comprises the stage of injecting or microinjecting into said capsules, cells, tissues, tissue parts, organs, organ parts, cell cores, non-human gametes and embryos at various stages of development, either freshly removed or appropriately preserved.

18. The process according to any of the claims 14 to 17 further comprising the incubation stage, in an appropriate culture medium, of said non-human immature female gametes.

19. The process according to any of the claims 14 to 18 further comprising the stage of extraction, purification, characterisation and sequencing of the substances produced such as hormones, metabolites, catabolites and other biologically active substances.

20. The process according to any of the claims 14 to 19 wherein, in step (a), said divalent or trivalent ion is calcium, barium, strontium, zinc, aluminium, iron or chromium chloride or sulphate at a concentration between 5 and 200 mmol/l.

21. The process according to any of the claims 14 to 20 wherein, in step (a), the culture medium used is selected from: physiological solution (isotonic saline), glucosate solution, Basal Medium Eagle (BME) and derivatives thereof, Hanks salts solution and derivatives thereof, tissue culture medium 199 (TCM 199) and derivatives thereof, phosphate buffered saline (PBS) and derivatives thereof, Krebs salts solution and derivatives thereof, Dulbecco modified Eagle's medium (DMEM) and derivatives thereof, tris-buffered medium (TBM) and derivatives thereof, Tyrode's salts solution and derivatives thereof, Modified sperm washing medium, modified human tubal fluid, Modified ham's F-10 medium, Upgraded B2 INRA medium, B2 INRA Menezo Medium, Upgraded B9 medium, optionally containing a biocompatible and/or biodegradable polymer which constitutes the artificial extracellular matrix.

22. The process according to any of the claims 14 to 21 wherein, in step (a), the medium used is TCM 199 and derivatives thereof, containing a hydrophilic polymer constituting the artificial extracellular matrix.

23. The process according to any of the claims 14 to 22 wherein, in step (a), the cellular sediment dilution /polymeric solution volume ratio is between 1:0.05 and 1:200.

24. The process according to claim 23 wherein the cellular sediment dilution / polymeric solution volume ratio is between 1:0.1 to 1:100.

25. The process according to any of the claims 14 to 24 wherein, in step (c), the extruded cellular suspension and the alginate solution volume ratio is between 1:1 and 1:250.

26. The process according to any of the claims 14 to 25 wherein, in step (c), extrusion occurs through the use of automated, semi-automated microencapsulators, peristaltic or piston pumps or alternatives, or by the use of manually operated syringes at such a speed as to produce from 10 to 250 drops/minute.

27. The process according to any of the claims 14 to 26 wherein, in step (c), the extrusion, with automated, semi-automated microencapsulators, peristaltic or piston pumps or alternatives, or by the use of manually operated syringes, occurs at such a speed as to produce 60 drops/minute.

28. The process according to any of the claims 14 to 27 wherein, in step (c), the extrusion of the cellular suspension occurs through the use of needles with internal diameters between 300 µm and 2000 µm.

29. The process according to any of the claims 14 to 28 wherein, in step (c), the alginate solution is kept stirring at a speed between 20 and 100 rpm and has a concentration between 0.1% and 1% w/v.

30. The process according to any of the claims 14 to 29 wherein, in step (c), the extruded cellular suspension and the alginate solution volume ratio is between 1:15 and 1:50.

31. The process according to any of the claims 14 to 30 wherein said alginates, in a 2% solution in water and at a temperature of 25°C, have a viscosity between 200 cP and 20000 cP.

32. The process according to any of the claims 14 to 31 wherein steps (a), (b) and (c) are performed at a temperature between 5°C and 40°C.

33. The process according to any of the claims 14 to 32 wherein steps (a), (b) and (c) are performed at a temperature between 20°C and 30°C.

34. The process according to any of the claims 14 to 33 wherein step (e) is performed at a temperature between 5°C and 40°C, for times between 1 minute and 120 minutes.

35. The process according to any of the claims 14 to 34 wherein step (e) is performed at a temperature between 20°C and 30°C, for times between 3 minutes and 30 minutes.

## Patentansprüche

1. Kapseln umfassend eine äußere Membran eines divalenten oder trivalenten Metallionensalzes von Alginsäure, und einen inneren Nukleus umfassend eine Suspension von nicht-humanen unreifen weiblichen Gameten und ein biokompatibles und/oder bioabbaubares hydrophiles Polymer, was eine künstliche extrazelluläre Matrix konstituiert, **dadurch gekennzeichnet, dass** das hydrophile Polymer Xanthan, welches eine Viskosität zwischen 800 und 1200 cP aufweist, ist, und **dadurch gekennzeichnet, dass** die Alginatmembran follikuläre Eierstockzellen enthält.

2. Kapseln gemäß Anspruch 1, wobei die Zellen in einem gelatineartigen Medium suspendiert sind.

3. Kapseln gemäß Ansprüchen 1 oder 2, **dadurch gekennzeichnet, dass** die nicht-humanen unreifen weiblichen Gameten und die follikulären Eierstockzellen *in vitro* in dreidimensionale Strukturen follikulärer Art auto-organisiert sind, was das *in vitro*-Wachstum von Geweben und multizellulären Strukturen, die funktionell ähnlich zu Organen sind, welche in dem gesamten Säugetierorganismus vorkommen, ermöglicht.

4. Kapseln nach einem der Ansprüche 1 bis 3, **dadurch gekennzeichnet, dass** die nicht-humanen unreifen weiblichen Gameten funktional ähnlich zu den Organen sind, welche in dem gesamten Organismus gefunden werden und in der Lage sind, Hormone wie Progesteron (P4) und 17β-Oestradiol (E2) und andere biologisch aktive Substanzen in Mengen zu synthetisieren und zu sekretieren, welche ähnlich sind zu denen, die diese Strukturen *in vivo* produzieren.

5. Kapseln nach einem der Ansprüche 1 bis 4, wobei die Alginatmembran gelatineartig, bioerodierbar ist und auf den inneren und/oder äußeren Oberflächen und/oder beiden Oberflächen quervernetzt ist.

6. Kapseln nach einem der Ansprüche 1 bis 5, **dadurch gekennzeichnet, dass** die Alginatmembran quervernetzt sein kann unter Verwendung von quervernetzenden Wirkstoffen ausgewählt aus: Protaminsulphat oder - phosphat, Poly-L-Lysinbromhydrat, Polyvinylamin oder Chitosanen.

7. Kapseln nach einem der Ansprüche 1 bis 6, wobei die äußere Membran sich aus divalenten Metallalginaten ausgewählt aus: Calcium, Barium, Strontium, Zink oder trivalenten Metallen ausgewählt aus: Aluminium, Eisen, Chrom, zusammensetzt.

8. Kapseln nach Anspruch 1, **dadurch gekennzeichnet, dass** das Xanthan in wässriger Lösung in Konzentrationen zwischen 0,01 % und 90 % des gesamten Kapselgewichts vorliegt.

9. Kapseln nach Anspruch 8, **dadurch gekennzeichnet, dass** das Xanthan in wässriger Lösung in Konzentrationen zwischen 0,5 % und 50 % des gesamten Kapselgewichts vorliegt.

10. Kapseln nach einem der Ansprüche 1 bis 9, **dadurch gekennzeichnet, dass** die Kapseln einen Durchmesser zwischen 0,5 mm und 30 mm haben, mit Membrandicken zwischen 300 µm und 5000 µm.

11. Kapseln nach Anspruch 10, **dadurch gekennzeichnet, dass** die Kapseln bevorzugt Durchmesser zwischen 2 mm und 10 mm haben.

12. Kapseln nach einem der Ansprüche 1 bis 11, **dadurch gekennzeichnet, dass** die Kapseln zwischen 5 mg und 200 mg wiegen.

13. Kapseln nach Anspruch 12, **dadurch gekennzeichnet, dass** die Kapseln bevorzugt zwischen 20 mg und 100 mg wiegen.

14. Ein Verfahren für die Herstellung von Kapseln gemäß einem der Ansprüche 1 bis 13, welches die folgenden Schritte umfasst:
a) Suspendieren von nicht-humanen unreifen weiblichen Gameten in einem Kulturmedium oder in einer geeigneten biologischen Flüssigkeit, welche ein biokompatibles und/oder bioabbaubares hydrophiles Polymer enthält, wobei das Polymer Xanthan ist;
b) Zugeben eines divalenten oder trivalenten Ionensalzes zu der so erhaltenen Suspension, bis Ionenkonzentrationen zwischen 1 und 500 mmol/l erreicht werden;
c) Extrudieren der zellulären Suspension durch Extruder, Mündungen, Düsen oder Nadeln, welche Dimension zwischen 50 µm und 5000 µm haben, in eine alkalische Metallalginatlösung im Kulturmedium, welche eine Konzentration zwischen 0,01 % und 5 % Gew./V aufweist, unter ständigem Rühren bei einer Geschwindigkeit zwischen 10 und 200 rpm;
d) Vehikularisieren einer zweiten zellulären Spezies bestehend aus follikulären Eierstockzellen, welche entweder frisch entfernt wurden oder auf geeignete Art und Weise aufbewahrt wurden, innerhalb der Alginatmembran, welche in Schritt c) erhalten wurde;
e) ggf. Quervernetzen der Kapseln, die so gebildet wurden, durch Grenzflächenpolymerisation des Alginats unter Verwendung der quervernetzenden Wirkstoffe gemäß Anspruch 5 bei einer Temperatur zwischen 5 °C und 40 °C für eine Zeitdauer zwischen 1 Minute und 120 Minuten.

15. Verfahren gemäß Anspruch 14, weiter umfassend die Rückgewinnungsphase der Kapseln durch Filtration, das Waschen derselben und ihre Suspendierung in Kulturmedium.

16. Verfahren nach Anspruch 14, weiter umfassend die Aufbewahrungsphase der Kapseln unter Laborkulturbedingungen, oder durch Lyophilisation, Kühlung, Einfrieren oder Cryokonservierung.

17. Verfahren nach Anspruch 14, wobei die Vehikularisierungsphase die Phase des Injizierens oder Mikro-Injiziierens in die Kapseln, Zellen, Gewebe, Gewebeteile, Organe, Organteile, Zellkerne, nicht-humane Gameten und Embryos in verschiedenen Entwicklungsphasen, welche entweder frisch entfernt wurden oder auf geeignete Art und Weise aufbewahrt wurden, umfasst.

18. Verfahren nach einem der Ansprüche 14 bis 17, weiter umfassend die Inkubationsphase in einem geeigneten Kulturmedium der nicht-humanen unreifen weiblichen Gameten.

19. Verfahren nach einem der Ansprüche 14 bis 18, weiter umfassend die Phase der Extraktion, Aufreinigung, Charakterisierung und Sequenzierung der Substanzen, welche produziert wurden, wie Hormone, Metaboliten, Cataboliten und andere biologisch aktive Substanzen.

20. Verfahren nach einem der Ansprüche 14 bis 19, wobei in Schritt (a) das divalente oder trivalente Ion Calcium, Barium, Strontium, Zink, Aluminium, Eisen oder Chromchlorid oder -sulphat bei einer Konzentration zwischen 5 und 200 mmol/l ist.

21. Verfahren nach einem der Ansprüche 14 bis 20, wobei in Schritt (a) das Kulturmedium, das verwendet wird, ausgewählt ist aus: physiologische Lösung (isotonische Kochsalzlösung), Glucoselösung, Basal-Medium Eagle (BME) und Derivate davon, Hanks-Salzlösungen und Derivate davon, Gewebekulturmedium 199 (TCM 199) und Derivate davon, Phosphat-gepufferte Kochsalzlösung (PBS) und Derivate davon, Krebs-Salzlösung und Derivate davon, Dulbecco-modifiziertes Eagle's-Medium (DMEM) und Derivate davon, dreifach gepuffertes Medium (TBM) und Derivate davon, Tyrode's-Salzlösung und Derivate davon, modifiziertes Spermawaschmedium, modifiziertes humanes Eileiterfluid, modifiziertes Ham's-F-10-Medium, verbessertes B2 INRA-Medium, B2 INRA Menezo-Medium, verbessertes B9-Medium, welche ggf. ein biokompatibles und/oder bioabbaubares Polymer enthalten, welches die künstliche extrazelluläre Matrix konstituiert.

22. Verfahren nach einem der Ansprüche 14 bis 21, wobei in Schritt (a) das Medium, welches verwendet wird, TCM 199 und Derivate davon ist, welches ein hydrophiles Polymer, welches die künstliche extrazelluläre Matrix konstituiert, enthält.

23. Verfahren nach einem der Ansprüche 14 bis 22, wobei in Schritt (a) das Verhältnis der zellulären Sedimentverdünnung/Volumen der polymeren Lösung zwischen 1:0,05 und 1:200 liegt.

24. Verfahren nach Anspruch 23, wobei das Verhältnis der zellulären Sedimentverdünnung/Volumen der polymeren Lösungs zwischen 1:0,1 bis 1:100 liegt.

25. Verfahren nach einem der Ansprüche 14 bis 24, wobei in Schritt (c) das Verhältnis zwischen extrudierter zellulärer Suspension und dem Alginatlösungsvolumen zwischen 1:1 und 1:250 liegt.

26. Verfahren nach einem der Ansprüche 14 bis 25, wobei in Schritt (c) die Extrusion durch die Verwendung eines automatisierten, halbautomatisierten Mikroenkapsulators, peristaltische oder Pumpkolben oder Alternativen, oder durch die Verwendung von manuell bedienten Spritzen bei so einer Geschwindigkeit stattfindet um von 10 bis 250 Tropfen/Minute zu produzieren.

27. Verfahren nach einem der Ansprüche 14 bis 26, wobei in Schritt (c) die Extrusion mit einem automatisierten, semi-automatisierten Mikroenkapsulator, peristaltischen oder Pumpkolben oder Alternativen, oder durch die Verwendung von manuell bedienten Spritzen bei so einer Geschwindigkeit stattfindet, dass 60 Tropfen/Minute produziert werden.

28. Verfahren nach einem der Ansprüche 14 bis 27, wobei in Schritt (c) die Extrusion der zellulären Suspension durch die Verwendung von Nadeln mit internen Durchmessern zwischen 300 µm und 2000 µm stattfindet.

29. Verfahren nach einem der Ansprüche 14 bis 28, wobei in Schritt (c) die Alginatlösung ständig bei einer Geschwindigkeit zwischen 20 und 100 rpm gerührt wird und eine Konzentration zwischen 0,1 % und 1 % Gew./V hat.

30. Verfahren nach einem der Ansprüche 14 bis 29, wobei in Schritt (c) das Verhältnis von der extrudierten zellulären Suspension und dem Alginatlösungsvolumen zwischen 1:15 und 1:50 liegt.

31. Verfahren nach einem der Ansprüche 14 bis 30, wobei die Alginate in einer 2 %-igen Lösung in Wasser und bei einer Temperatur von 25 °C eine Viskosität zwischen 200 cP und 20000 cP haben.

32. Verfahren nach einem der Ansprüche 14 bis 31, wobei Schritte (a), (b) und (c) bei einer Temperatur zwischen 5 °C und 40 °C durchgeführt werden.

33. Verfahren nach einem der Ansprüche 14 bis 32, wobei die Schritte (a), (b) und (c) bei einer Temperatur zwischen 20 °C und 30 °C durchgeführt werden.

34. Verfahren nach einem der Ansprüche 14 bis 33, wobei Schritt (e) bei einer Temperatur zwischen 5 °C und 40 °C für Zeitspannen zwischen 1 Minute und 120 Minuten durchgeführt wird.

35. Verfahren nach einem der Ansprüche 14 bis 34, wobei Schritt (e) bei einer Temperatur zwischen 20 °C und 30 °C für Zeitspannen zwischen 3 Minuten und 30 Minuten durchgeführt wird.

## Revendications

1. Capsules comprenant une membrane externe d'un sel d'ion métallique bivalent ou trivalent de l'acide alginique, et un noyau interne comprenant une suspension de gamètes femelles immatures non humains et d'un polymère hydrophile biocompatible et/ou biodégradable, constituant une matrice extracellulaire artificielle, **caractérisées en ce que** le polymère hydrophile est de la gomme xanthane présentant une viscosité entre 800 et 1200 cP et **en ce que** la membrane d'alginate contient des cellules folliculaires ovariennes.

2. Capsules selon la revendication 1, dans lesquelles lesdites cellules sont en suspension dans un milieu gélatineux.

3. Capsules selon les revendications 1 ou 2, **caractérisées en ce que** lesdits gamètes femelles immatures non humains et lesdites cellules folliculaires ovariennes sont auto-organisés *in vitro* en structures tridimensionnelles de type folliculaire, qui permettent la croissance *in vitro* de tissus et de structures multicellulaires fonctionnellement similaires à des organes présents dans l'organisme entier de mammifère.

4. Capsules selon l'une quelconque des revendications 1 à 3, **caractérisées en ce que** lesdits gamètes femelles immatures non humains sont fonctionnellement similaires aux organes trouvés dans l'organisme entier et sont capables de synthétiser et de sécréter des hormones telles que la progestérone (P4) et le 17β-oestradiol (E2) et d'autres substances biologiquement actives dans des quantités similaires à celles que lesdites structures produisent *in vivo.*

5. Capsules selon l'une quelconque des revendications 1 à 4, dans lesquelles ladite membrane d'alginate est gélatineuse, bioérodable et est réticulée sur les surfaces internes et/ou externes et/ou sur les deux surfaces.

6. Capsules selon l'une quelconque des revendications 1 à 5, **caractérisées en ce que** la membrane d'alginate peut être réticulée en utilisant des agents de réticulation choisis parmi : le sulfate ou le phosphate de protamine, le bromhydrate de poly-L-lysine, la polyvinylamine ou les chitosanes.

7. Capsules selon l'une quelconque des revendications 1 à 6, dans lesquelles la membrane externe est constituée par des alginates de métaux bivalents choisis parmi : le calcium, le baryum, le strontium, le zinc ou de métaux trivalents choisis parmi : l'aluminium, le fer, le chrome.

8. Capsules selon la revendication 1, **caractérisées en ce que** ladite gomme xanthane, en solution aqueuse, est présente dans des concentrations entre 0,01 % et 90 % du poids total de la capsule.

9. Capsules selon la revendication 8, **caractérisées en ce que** la gomme xanthane, en solution aqueuse, est présente dans des concentrations entre 0,5 % et 50 % du poids total de la capsule.

10. Capsules selon l'une quelconque des revendications 1 à 9, **caractérisées en ce que** lesdites capsules présentent des diamètres entre 0,5 mm et 30 mm, avec des épaisseurs de membrane entre 300 µm et 5000 µm.

11. Capsules selon la revendication 10, **caractérisées en ce que** lesdites capsules présentent de préférence des diamètres entre 2 mm et 10 mm.

12. Capsules selon l'une quelconque des revendications 1 à 11, **caractérisées en ce que** lesdites capsules pèsent entre 5 mg et 200 mg.

13. Capsules selon la revendication 12, **caractérisées en ce que** lesdites capsules pèsent de préférence entre 20 mg et 100 mg.

14. Procédé de préparation des capsules selon l'une quelconque des revendications 1 à 13, qui comprend les étapes suivantes :
a) la mise en suspension de gamètes femelles immatures non humains dans un milieu de culture ou dans un liquide biologique approprié contenant un polymère hydrophile biocompatible et/ou biodégradable, ledit polymère étant de la gomme xanthane ;
b) l'addition à la suspension ainsi obtenue d'un sel d'ion bivalent ou trivalent jusqu'à l'obtention de concentrations en ions entre 1 et 500 mmol/l ;
c) l'extrusion de la suspension cellulaire à travers des extrudeurs, des orifices, des embouts ou des aiguilles ayant des dimensions entre 50 µm et 5000 µm dans une solution d'alginate de métal alcalin dans le milieu de culture, ayant une concentration entre 0,01 % et 5 % p/v, en poursuivant l'agitation à une vitesse entre 10 et 200 tr/min ;
d) la véhicularisation au sein de la membrane d'alginate obtenue dans l'étape c) d'une seconde espèce cellulaire constituée de cellules folliculaires ovariennes soit fraîchement prélevées soit conservées de manière appropriée ;
e) éventuellement, la réticulation des capsules ainsi formées, par l'intermédiaire d'une polymérisation interfaciale de l'alginate en utilisant les agents de réticulation selon la revendication 5, à une température entre 5 °C et 40 °C pendant un temps entre 1 minute et 120 minutes.

15. Procédé selon la revendication 14, comprenant en outre l'étape de récupération des capsules par filtration, le lavage de celles-ci et leur suspension dans le milieu de culture.

16. Procédé selon la revendication 14, comprenant en outre l'étape de conservation des capsules dans des conditions de culture en laboratoire, ou par lyophilisation, réfrigération, congélation ou cryoconservation.

17. Procédé selon la revendication 14, dans lequel ladite étape de véhicularisation comprend l'étape d'injection ou de micro-injection dans lesdites capsules, de cellules, de tissus, de parties de tissus, d'organes, de parties d'organes, de noyaux cellulaires, de gamètes et d'embryons non humains à divers stades du développement, soit fraîchement prélevés soit conservés de manière appropriée.

18. Procédé selon l'une quelconque des revendications 14 à 17, comprenant en outre l'étape d'incubation, dans un milieu de culture approprié, desdits gamètes femelles immatures non humains.

19. Procédé selon l'une quelconque des revendications 14 à 18, comprenant en outre l'étape d'extraction, de purification, de caractérisation et de séquençage des substances produites comme des hormones, des métabolites, des catabolites et d'autres substances biologiquement actives.

20. Procédé selon l'une quelconque des revendications 14 à 19, dans lequel, dans l'étape (a), ledit ion bivalent ou trivalent est le chlorure ou le sulfate de calcium, de baryum, de strontium, de zinc, d'aluminium, de fer ou de chrome à une concentration entre 5 et 200 mmol/l.

21. Procédé selon l'une quelconque des revendications 14 à 20, dans lequel, dans l'étape (a), le milieu de culture utilisé est choisi parmi : une solution physiologique (solution saline isotonique), une solution de glucosate, le milieu basal de Eagle (BME) et ses dérivés, la solution des sels de Hanks et ses dérivés, le milieu de culture tissulaire 199 (TCM 199) et ses dérivés, la solution tampon phosphate (PBS) et ses dérivés, la solution des sels de Krebs et ses dérivés, le milieu de Eagle modifié par Dulbecco (DMEM) et ses dérivés, le milieu tamponné par du Tris (TBM) et ses dérivés, la solution des sels de Tyrode et ses dérivés, le milieu de lavage des spermatozoïdes modifié, le liquide tubaire humain modifié, le milieu F10 de Ham modifié, le milieu Upgraded B2 INRA, le milieu B2 INRA Menezo, le milieu Upgraded B9, contenant éventuellement un polymère biocompatible et/ou biodégradable qui constitue la matrice extracellulaire artificielle.

22. Procédé selon l'une quelconque des revendications 14 à 21, dans lequel, dans l'étape (a), le milieu utilisé est le TCM 199 et ses dérivés, contenant un polymère hydrophile constituant la matrice extracellulaire artificielle.

23. Procédé selon l'une quelconque des revendications 14 à 22, dans lequel, dans l'étape (a), le rapport de la dilution du sédiment cellulaire/le volume de la solution polymère se situe entre 1/0,05 et 1/200.

24. Procédé selon la revendication 23, dans lequel le rapport de la dilution du sédiment cellulaire/le volume de la solution polymère se situe entre 1/0,1 et 1/100.

25. Procédé selon l'une quelconque des revendications 14 à 24, dans lequel, dans l'étape (c), le rapport de la suspension cellulaire extrudée et du volume de la solution d'alginate se situe entre 1/1 et 1/250.

26. Procédé selon l'une quelconque des revendications 14 à 25, dans lequel, dans l'étape (c), l'extrusion se produit grâce à l'utilisation de micro-encapsulateurs automatisés ou semi-automatisés, de pompes péristaltiques ou à piston ou de variantes, ou par l'utilisation de seringues à fonctionnement manuel, à une vitesse permettant de produire de 10 à 250 gouttes/minute.

27. Procédé selon l'une quelconque des revendications 14 à 26, dans lequel, dans l'étape (c), l'extrusion avec des micro-encapsulateurs automatisés ou semi-automatisés, des pompes péristaltiques ou à piston ou des variantes, ou par l'utilisation de seringues à fonctionnement manuel, se produit à une vitesse permettant de produire 60 gouttes/minute.

28. Procédé selon l'une quelconque des revendications 14 à 27, dans lequel, dans l'étape (c), l'extrusion de la suspension cellulaire se produit grâce à l'utilisation d'aiguilles avec des diamètres internes compris entre 300 µm et 2000 µm.

29. Procédé selon l'une quelconque des revendications 14 à 28, dans lequel, dans l'étape (c), la solution d'alginate est maintenue sous agitation à une vitesse entre 20 et 100 tr/min et présente une concentration entre 0,1 % et 1 % p/v.

30. Procédé selon l'une quelconque des revendications 14 à 29, dans lequel, dans l'étape (c), le rapport de la suspension cellulaire extrudée et du volume de la solution d'alginate se situe entre 1/15 et 1/50.

31. Procédé selon l'une quelconque des revendications 14 à 30, dans lequel lesdits alginates, dans une solution à 2 % dans de l'eau et à une température de 25 °C, présentent une viscosité entre 200 cP et 20000 cP.

32. Procédé selon l'une quelconque des revendications 14 à 31, dans lequel les étapes (a), (b) et (c) sont réalisées à une température entre 5 °C et 40 °C.

33. Procédé selon l'une quelconque des revendications 14 à 32, dans lequel les étapes (a), (b) et (c) sont réalisées à une température entre 20 °C et 30 °C.

34. Procédé selon l'une quelconque des revendications 14 à 33, dans lequel l'étape (e) est réalisée à une température entre 5 °C et 40 °C, pendant des temps entre 1 minute et 120 minutes.

35. Procédé selon l'une quelconque des revendications 14 à 34, dans lequel l'étape (e) est réalisée à une température entre 20 °C et 30 °C, pendant des temps entre 3 minutes et 30 minutes.
